# EUROPEAN PATENT SPECIFICATION

(11) **EP 2 740 349 B1**
(45) Date of publication and mention of the grant of the patent: **26.02.2020**
(21) Application number: 12822667.7
(22) Date of filing: 27.03.2012
(51) Int. Cl.: A01G 33/00, A01G 7/04, A01H 3/02, C12N 1/12, C12N 13/00

(54) **ALGAE CULTIVATION METHOD**
ALGENANBAUVERFAHREN
PROCÉDÉ DE CULTURE D'ALGUES

(30) Priority: 05.08.2011 JP 2011172089
(43) Date of publication of application: 11.06.2014
(73) Proprietor: Showa Denko K.K., Tokyo 105-8518 (JP); Yamaguchi University, Yamaguchi 753-8511 (JP)
(72) Inventor: SHIGYO, Masayoshi, Yamaguchi-shi Yamaguchi 753-8515 (JP); SUZUKI, Hiroshi, Tokyo 105-8518 (JP); YAMAUCHI, Naoki, Yamaguchi-shi Yamaguchi 753-8515 (JP); ARA, Hironori, Tokyo 105-8518 (JP); SHIMOKAWA, Akihiro, Yamaguchi-shi Yamaguchi 753-8515 (JP); MATSUMOTO, Misato, Yamaguchi-shi Yamaguchi 753-8515 (JP); TONOOKA, Yuki, Ube-shi Yamaguchi 755-8505 (JP)
(74) Representative: Strehl Schübel-Hopf & Partner
(86) International application number: PCT/JP2012/057853
(87) International publication number: WO 2013/021675

(56) References cited:
- EP-A1- 2 025 220
- JP-A- 8 103 167
- JP-A- 11 266 727
- JP-A- 11 266 727
- US-A- 3 930 335
- R.M. FORSTER ET AL: "Influence of blue light on the photosynthetic capacity of marine plants from different taxonomic, ecological and morphological groups", EUROPEAN JOURNAL OF PHYCOLOGY, vol. 29, no. 1, 17 February 1994 (1994-02-17), pages 21-27, XP055576450, DE ISSN: 0967-0262, DOI: 10.1080/09670269400650441

## Description

### Technical Field

The present invention relates to an algae cultivation method. More particularly, it relates to an algae cultivation method for promoting the proliferation by irradiating an artificial light to algae.

### Background Art

Conventionally, plant culture methods involve a technology for promoting a seedling growth by irradiating an artificial light to the seedling of a plant. By promoting the growth of the plant, the culture period can be shortened and the number of cropping in an identical place can be increased. Moreover, the quantity of the crops can be increased even within the same culture period if the plant can be grown to a larger size.

As a plant culture method utilizing an artificial light irradiation, a plant irradiation equipment comprising an alternate irradiation of a green light and a white light to a plant is disclosed for example in Patent Document 1. This irradiation equipment establishes the daytime-to-nighttime variation by irradiating the green light of a wavelength of 500 to 570 nm and the white light of a wavelength of 300 to 800 nm alternately, thereby facilitating the translocation effect of the plant while aiming at growing the plant.

Also for example in Patent Document 2, a light source for plant culture which irradiates a light energy for cultivation, growth, culture, and tissue cultivation by simultaneous or alternate turning on of a light emitting diode emitting a blue light (400 to 480 nm) and a light emitting diode emitting a red light (620 to 700 nm) is disclosed. This light source for plant culture intends to culture the plant at a high energy efficiency by irradiating the lights only of the wavelengths in agreement with the chlorophyll's light absorption peaks (around 450 nm and around 660 nm).

In Patent Document 2, it is prescribed that the blue light and the red light may be irradiated simultaneously or irradiated alternately (see "Claim 1" in the relevant document). In Patent Document 2, however, it is just described, when comparing the irradiation only with the blue light, the irradiation only with the red light, and the simultaneous irradiation with the blue light and the red light, that, under the simultaneous irradiation, a healthy growth similar to that observed in a culture under a solar light (compared with a non-healthy growth such as succulent growth observed under a single light irradiation) was observed (see Paragraph [0011] in the relevant document), and no growth promoting effect under the alternate irradiation with the blue light and the red light was identified. Accordingly, Patent Document 2 contains substantially no disclosure of a plant culture method by an alternate irradiation with a blue light and a red light.

On the other hand, organisms undergoing photosynthesis other than higher plants growing on the ground are algae. Generally, algae include a large number of unicellular or multicellular organism species belonging to prokaryotes and eukaryotes, to which diatoms, dinoflagellates and green algae belong. Among these, the green algae include those being hopeful as starting materials for biological fuels because of their ability of fixing carbon dioxide by photosynthesis to produce hydrocarbons capable of serving as petroleum substitutes and those used as starting materials for health foods and pharmaceuticals because of their ability of producing a large amount of nutritional components and antioxidant substances.

For example, Patent Document 3 describes a method for recovering hydrocarbons from cultivated green algae. Patent Document 4 also discloses green algae which produce astaxanthin which is one of red carotinoids and has a potent antioxidative effect. Patent Document 5 provides a method for culturing algae capable of controlling a supply rate of the algae.

### Citation List

### Patent Literatures

[Patent Document 1] Japanese Unexamined Patent Application Publication No. H06-276858
[Patent Document 2] Japanese Unexamined Patent Application Publication No. H08-103167
[Patent Document 3] Japanese Unexamined Patent Application Publication No. 2010-252700
[Patent Document 4] Japanese Unexamined Patent Application Publication No. 2007-097584
[Patent Document 5] JP 11 266727 A

### Summary of Invention

### Technical Problem

The algae are produced as starting materials for biological fuels, health foods, and pharmaceuticals industrially by a large scale cultivation (see aforementioned Patent Documents 3 and 4). In the industrial cultivation of algae, it is demanded to promote the algae proliferation to shorten the cultivation period thereby increasing the producibility. Under such a circumstance, an object of the present invention is majorly to provide a convenient method for promoting the proliferation of algae.

### Solution to Problem

As a result of our intensive study on the algae proliferation promoting effect of an artificial light irradiation, we surprisingly discovered that an extremely remarkable effect can be obtained by a method as simple as an alternate irradiation of a red light and a blue light.

Based on these findings, the present invention provides an algae cultivation method for promoting the proliferation of algae by conducting a procedure for irradiating a red illuminative light to the algae and a procedure for irradiating a blue illuminative light to the algae separately and independently of each other within a certain time period.

In particular, in this algae cultivation method (Shigyo Method), the procedure for irradiating the red illuminative light and the procedure for irradiating the blue illuminative light are conducted alternately and successively.
The present invention provides an algae cultivation method according to the appended claims.

The present description also discloses an algae cultivation equipment comprising: a light irradiation part for irradiating a red illuminative light and a blue illuminative light to the algae; and a controlling part for controlling the light irradiation part to conduct a step for irradiating the red illuminative light to the algae and a step for irradiating the blue illuminative light to the algae separately and independently of each other within a certain time period.

In this algae cultivation equipment, the aforementioned controlling part allows the light quantities, wavelengths, and/or irradiation times of the aforementioned red illuminative light and the aforementioned blue illuminative light irradiated from the aforementioned light irradiation part to be kept at certain values or to be varied in certain patterns. It is preferable that the aforementioned light irradiation part comprises light emitting diodes which emit a red light or a blue light.

In the present invention, "the algae" include a wide range of unicellular organisms such as green algae, brown algae, blue-green algae, purple photosynthetic bacteria and the like and aquatic multicellular organisms having photosynthetic ability such as waterweeds, regardless of prokaryotes or eukaryotes.

### Advantageous Effects of Invention

According to the present invention, an algae cultivation method which is convenient and capable of achieving an excellent proliferation promoting effect is provided.

### Brief Description of Drawings

Fig. 1 is a schematic view illustrating the procedure of the algae cultivation method according to the first embodiment of the present invention.
Fig. 2 is a schematic view illustrating the procedure of the algae cultivation method according to the second embodiment of the present invention (not belonging to the scope of the present invention).
Fig. 3 is a schematic view illustrating the procedure of the algae cultivation method according to the third embodiment of the present invention (not belonging to the scope of the present invention).
Fig. 4 is a drawing-substituting photograph showing the results of the investigation of the proliferation promoting effect of the alternate irradiation with the red light and the blue light in Botryococcus braunii (Test Example 1).
Fig. 5 is a drawing-substituting graph showing the results of the investigation of the proliferation promoting effect of the alternate irradiation with the red light and the blue light in Botryococcus braunii (Test Example 1).
Fig. 6 is a drawing-substituting graph showing the results of the investigation of the proliferation promoting effect of the alternate irradiation with the red light and the blue light in Botryococcus braunii (Test Example 1).
Fig. 7 is a drawing-substituting graph showing the results of the investigation of the proliferation promoting effect of the alternate irradiation with the red light and the blue light in Chlorella kessleri (Test Example 2).
Fig. 8 is a drawing-substituting graph showing the results of the investigation of the proliferation promoting effect of the alternate irradiation with the red light and the blue light in Hematococcus lacustris (Test Example 3).
Fig. 9 is a drawing-substituting graph showing the results of the investigation of the proliferation promoting effect of the alternate irradiation with the red light and the blue light in Hematococcus lacustris (Test Example 3).

### Description of Embodiments

The preferred embodiments of the present invention are described below with referring to the drawings. The following embodiments are the examples of the representative of the embodiments of the present invention which do not serve to allow the scope of the invention to be interpreted narrowly. The description is made in the order shown below.
1. Algae cultivation method
   (1) Algae cultivation method according to first embodiment.
   (2) Algae cultivation method according to second embodiment (not belonging to the scope of the present invention).
   (3) Algae cultivation method according to third embodiment (not belonging to the scope of the present invention).
   (4) Wavelength
   (5) Light quantity
   (6) Irradiation time
2. Algae cultivation equipment
   (1) Algae cultivation equipment used in the first embodiment
      (1-1) Light irradiation part
      (1-2) Controlling part
3. Cultivated algae

### 1. Algae cultivation method

### (1) Algae cultivation method according to first embodiment

The algae cultivation method according to the present invention is a method for promoting the proliferation of algae by conducting a procedure for irradiating a red illuminative light to the algae (hereinafter referred to also as "red light irradiation step ") and a procedure for irradiating a blue illuminative light to the algae (hereinafter referred to also as "blue light irradiation step") separately and independently of each other within a certain time period.

The red illuminative light is a red light having a wavelength range substantially of 570 to 730 nm. The red illuminative light may contain a light having a wavelength range different from that of the aforementioned red light, but preferably contains no blue light described below. The red illuminative light contains only the aforementioned red light in an especially preferred case. The blue illuminative light is a blue light having a wavelength range substantially of 400 to 515 nm. The blue illuminative light may contain a light having a wavelength range different from that of the aforementioned blue light, but preferably contains no red light described above. The blue illuminative light contains only the aforementioned blue light in an especially preferred case. Moreover, the red illuminative light contains no aforementioned blue light and the blue illuminative light contains no aforementioned red light in a preferred case, and the red illuminative light is exclusively the aforementioned red light and the blue illuminative light is exclusively the aforementioned blue light in an especially preferred case.

As used herein, "a certain time period" means a period of any length of the time during the algae cultivation. This period may be as long as the entire cultivation period. The shortest period may be set as desired as long as the effect of the invention can be exerted. This period may employ, for example an hour (h) as a time length unit, or may employ a longer time length unit (for example, day (D)) or a shorter time length unit (for example, minute (min)).

The algae cultivation method according to the present invention can be started or ended at any time during the course of the algae cultivation, and can be applied over any time period.

The phrase "separately and independently of each other" means that the red light irradiation step and the blue light irradiation step are present separately during the aforementioned period. It is sufficient that at least each one step of the red light irradiation step and the blue light irradiation step is included in the aforementioned period.

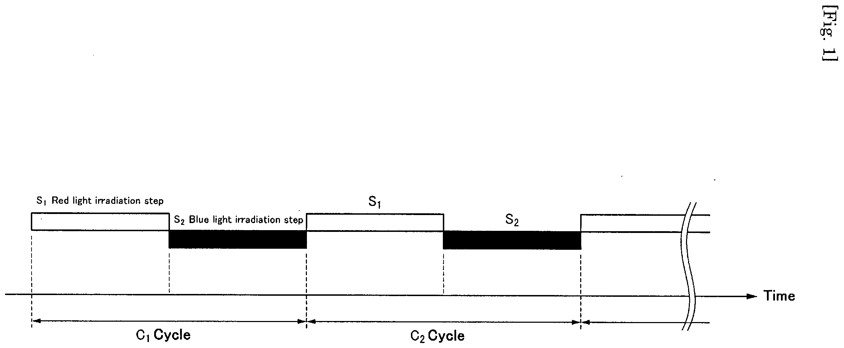

The red light irradiation step and the blue light irradiation step are conducted alternately and successively by allowing the both steps to be intervened by a procedure for irradiating the red illuminative light and the blue illuminative light simultaneously to algae or by a procedure for interrupting the irradiation of the light to the algae. They are conducted alternately and successively for the purpose of enhancing the algae proliferation promoting effect. The embodiments of the algae cultivation method according to the present invention are described in detail with referring to Fig. 1 to Fig. 3. It is also possible as a matter of course to conduct the plant culture method according to the present invention while combining the respective embodiments illustrated in Fig. 1 to Fig. 3 with each other as appropriate.

Fig. 1 is a schematic view illustrating the procedure of the algae cultivation method according to the first embodiment of the present invention. This embodiment conducts the red light irradiation step and the blue light irradiation step alternately and successively.

In the figure, the symbol S₁ represents the red light irradiation step, and the symbol S₂ represents the blue light irradiation step. In this embodiment, the red light irradiation step S₁ and the blue light irradiation step S₂ are conducted alternately and successively, and the irradiation cycle consisting of the red light irradiation step S₁ and the blue light irradiation step S₂ is conducted repeatedly.

By irradiating the red illuminative light and the blue illuminative light alternately to the algae as described above, the division can markedly be promoted (see Examples described below).

While the case in which the procedure is started from the red light irradiation step S₁ in the first irradiation cycle C₁ is exemplified here, any of the red light irradiation step S₁ and the blue light irradiation step S₂ may be conducted earlier as desired in each irradiation cycle.

### (2) Algae cultivation method according to second embodiment (not belonging to the scope of the present invention)

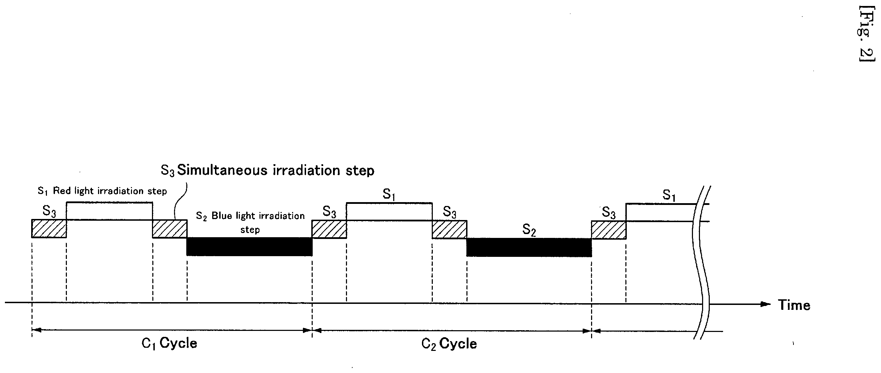

Fig. 2 is a schematic view illustrating the procedure of the algae cultivation method according to the second embodiment (not belonging to the scope of the present invention). This embodiment conducts the red light irradiation step and the blue light irradiation step intermittently and repeatedly by allowing the both steps to be intervened by a procedure for irradiating the red illuminative light and the blue illuminative light simultaneously to algae (hereinafter referred to also as "simultaneous irradiation step").

In the figure, the symbol S₃ represents the simultaneous irradiation step. In this embodiment, the red light irradiation step S₁ and the blue light irradiation step S₂ are conducted intermittently while being intervened by the simultaneous irradiation step S₃, and the irradiation cycle consisting of the red light irradiation step S₁, simultaneous irradiation step S₃, and blue light irradiation step S₂ is conducted repeatedly.

While the case in which the procedure is started from the simultaneous irradiation step S₃ in the first irradiation cycle C₁ is exemplified here, any of the red light irradiation step S₁, simultaneous irradiation step S₃, and blue light irradiation step S₂ may be conducted earlier as desired in each irradiation cycle.

### (3) Algae cultivation method according to third embodiment (not belonging to the scope of the present invention)

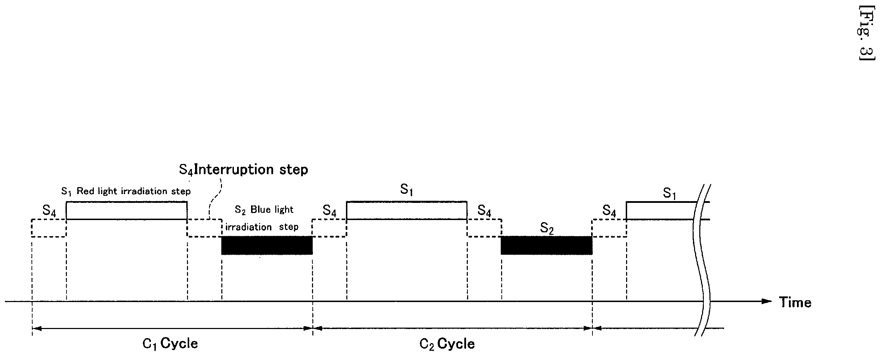

Fig. 3 is a schematic view illustrating the procedure of the algae cultivation method according to the third embodiment (not belonging to the scope of the present invention). This embodiment conducts the red light irradiation step and the blue light irradiation step intermittently and repeatedly by allowing the both steps to be intervened by a procedure for interrupting the irradiation of the light to the algae (hereinafter referred to also as "interruption step").

In the figure, the symbol S₄ represents the interruption step. In this embodiment, the red light irradiation step S₁ and the blue light irradiation step S₂ are conducted intermittently while being intervened by the interruption step S₄, and the irradiation cycle consisting of the red light irradiation step S₁, the interruption step S₄, and the blue light irradiation step S₂ is conducted repeatedly.

While the case in which the procedure is started from the interruption step S₄ in the first irradiation cycle C₁ is exemplified here, any of the red light irradiation step S₁, interruption step S₄, and blue light irradiation step S₂ may be conducted earlier as desired in each irradiation cycle.

### (4) Wavelength

In the algae cultivation method according to each embodiment described above, the red light is a light having a wavelength of 570 to 730 nm, and the light having a wavelength of 635 to 660 nm as the center wavelength is employed preferably. On the other hand, the blue light is a light having a wavelength of 400 to 515 nm, and the light having a wavelength of 450 nm as the center wavelength is employed preferably. The red light and the blue light may be those having a certain wavelength range whose center wavelengths are the aforementioned wavelengths. The wavelength range for example of the blue light is 450 ± 30 nm, preferably 450 ± 20 nm, more preferably 450 ± 10nm.

The wavelength of the red light and the blue light may vary within the aforementioned wavelength range, and it is possible to change the wavelength for example in the Nth (N is an integer of 1 or more) irradiation cycle C_{N}. It is also possible that the wavelength may be different between the Nth irradiation cycle C_{N} and the Mth (M is an integer of 1 or more which is different from N) irradiation cycle C_{M} within the aforementioned wavelength range.

Furthermore, it is also possible that, in the aforementioned red light irradiation step S₁, simultaneous irradiation step S₃, and blue light irradiation step S₂, the red light and the blue light may be combined with lights having other wavelength ranges to conduct an irradiation with the lights having several wavelength ranges.

### (5) Light quantity (intensity)

While the light quantities (intensities) of the red light and the blue light in the red light irradiation step S₁, the blue light irradiation step S₂, and the simultaneous irradiation step S₃ are not limited particularly, each, when expressed for example as a photosynthetic photon flux density (PPFD), is approximately 1 to 1000 µmol/m²s, preferably 10 to 500 µmol/m²s, more preferably 50 to 250 µmol/m²s.

The light quantity (intensity) ratio of the red illuminative light and the blue illuminative light in the aforementioned each step may be set as desired for example to a ratio of "red:blue" or "blue:red" of 1:1, 5:3, 2:1, 3:1, 4:1, 10:1, 20:1, and the like.

The light quantity of the red illuminative light and the blue illuminative light may vary within the aforementioned range, and it is possible to change the intensity for example in the Nth (N is an integer of 1 or more) irradiation cycle C_{N}. It is also possible that the light intensity may be differentiated between the Nth irradiation cycle C_{N} and the Mth (M is an integer of 1 or more which is different from N) irradiation cycle C_{M} within the aforementioned range.

### (6) Irradiation time

In the algae cultivation method according to aforementioned each embodiment, the time period of a single irradiation cycle is the entire cultivation period at maximum. The shortest period may be set as desired as long as the effect of the invention can be exerted. The time period of a single irradiation cycle may employ, for example an hour (h) as a time length unit, or may employ a longer time length unit (for example, day (D)) or a shorter time length unit (for example, minute (min)).

For example, in the algae cultivation method according to the first embodiment wherein the red light irradiation step S₁ and the blue light irradiation step S₂ are conducted alternately and successively, if the single irradiation cycle takes a single day, then the red light irradiation step S₁ may take 12 hours and the blue light irradiation step S₂ may take 12 hours. Also for example, if the irradiation cycle is repeated 4 times a day, then the single irradiation cycle takes 6 hours, and the red light irradiation step S₁ may take 3 hours and the blue light irradiation step S₂ may take 3 hours.

The time period of a single irradiation cycle may vary between the Nth irradiation cycle C_{N} and the Mth (M is an integer of 1 or more which is different from N) irradiation cycle C_{M}. For example, the irradiation cycle C_{N} may take 12 hours and the subsequent irradiation cycle C_{N+1} may take 6 hours.

The ratio of the time periods of the red light irradiation step S₁, blue light irradiation step S₂, simultaneous irradiation step S₃, and interruption step S₄ within a single irradiation cycle may be set as desired. In the algae cultivation method according, for example, to the aforementioned first embodiment, if a single irradiation cycle takes a single day, then "the red light irradiation step S₁/the blue light irradiation step S₂" may be set as desired for example to "12 hours/12 hours (1:1)", "16 hours/8 hours (2:1)", "21 hours/3 hours (7:1)" and the like.

Most preferably, in the plant culture method according to the first embodiment where the red light irradiation step S₁ and the blue light irradiation step S₂ are conducted alternately and successively, the red light irradiation step S₁ and the blue light irradiation step S₂ are switched to each other at a time interval suited to the cell division cycle of the algae.

### (7) Others

In the algae cultivation method according to the present invention, the cultivation conditions other than the illumination conditions may be similar to those in any known cultivation methods. For example, the culture medium may be a culture medium for fresh water algae (such as AF6 culture medium, C culture medium, URO culture medium, and the like), a culture medium for marine algae (ESM culture medium, f/2 culture medium, IMR culture medium, MNK culture medium, and the like).

The algae cultivation method according to the present invention is considered to exert a remarkable division promoting effect by allowing the irradiation with the red light and the blue light to act in harmony with the photosynthesis mechanism of the algae. In the algae cultivation method according to the present invention, the proliferation promoting effect can further be enhanced when combined with the use of carbon dioxide gas or known agents having photosynthesis promoting effects.

### 2. Algae cultivation equipment

### (1) Algae cultivation equipment used in the to first embodiment

### (1-1) Light irradiation part

The algae cultivation equipment used in the present invention can perform each procedure of the aforementioned algae cultivation method, and comprises a light irradiation part for irradiating a red illuminative light and a blue illuminative light to the algae; and a controlling part for controlling the light irradiation part to conduct a step for irradiating the red illuminative light to the algae and a step for irradiating the blue illuminative light to the algae separately and independently of each other within a certain time period.

The light irradiation part comprises light sources which emit the red light and the blue light. The light sources of the red light and the blue light may be known light sources. A light source which is employed preferably is a photosemiconductor device such as a light emitting diode (LED) or a laser diode (LD) which allows the wavelength to be selected easily and emits a light having a high proportion of the photo energy of the valid wavelength range. When using an electroluminescence (EL), the EL may be organic or inorganic.

The photosemiconductor device is compact-sized, long-lived, and capable of emitting at a specific wavelength depending on the material with no unnecessary heat emission thereby achieving a favorable energy efficiency, and hardly impairs the cells of algae even with the irradiation from a close proximity. As a result, by using the photosemiconductor device as a light source, it becomes possible to conduct the culture at a lower electric power cost in a smaller space when compared with other light source.

The light source may be an SMD line light source in which SMDs each having a combination of a single red photosemiconductor device and a single blue photosemiconductor device mounted thereon (2 Chips Surface Mount Device) are aligned linearly, or a single color line light source or a single color panel light source in which only either one of the red photosemiconductor devices or the blue photosemiconductor devices are aligned linearly or planarly.

The semiconductor device is capable, in principle, of flicker operation at a frequency as high as several megahertz (MHz) or higher. Accordingly, by using the photosemiconductor device as a light source, the red light irradiation step S₁, the blue light irradiation step S₂, the simultaneous irradiation step S₃, and the interruption step S₄ can be switched to each other very quickly.

As LEDs emitting lights having the aforementioned wavelength ranges, a red LED such as an aluminum/gallium/indium/phosphorus-based light emitting diode (gallium/phosphorus-based board, red wavelength: 660 nm) marketed under a product number of HRP-350F by Showa Denko K.K. and a blue LED such as a light emitting diode having a product number of GM2LR450G of the aforementioned company are exemplified.

The light sources of the light emitting diodes may for example be tubular and compact fluorescent lamps, and bulbar fluorescent lamps, high intensity discharge lamps, metal halide lamps, laser diodes, and the like. In combination with these light sources, an optical filter for selective use of the light in the aforementioned wavelength range may be employed.

### (1-2) Controlling part

The controlling part allows the light quantity (intensity), wavelength, and/or irradiation time of the red illuminative light and the blue illuminative light irradiated from the light irradiation part to be kept at certain values or to be varied in certain patterns.

The controlling part can be constructed using a versatile computer. When using, for example, an LED as a light source, the controlling part serves, based on the controlling pattern stored or memorized preliminarily in a memory or a hard disc, to adjust the level of the LED operation current and alter the intensity and the irradiation time of the red illuminative light and the blue illuminative light. In addition, the controlling part serves, based on the controlling pattern, to operate several LEDs emitting the lights in different wavelength ranges while switching them to each other, thereby altering the wavelength range of the irradiated light.

### 3. Cultivated algae

The algae targeted by the algae cultivation method according to the present invention
belong to Chlorophyceae (Class Chlorophyceae). Chlorophyceae includes green algae of the genera of Botryococcus, Hematococcus, and Chlorella,

Botryococcus braunii as a species of the genus Botryococcus and Pseudochoricystis ellipsoidea as a species of the genus Pseudochoricystis undergo photosynthesis to fix carbon dioxide to produce hydrocarbons capable of serving as petroleum (heavy oil or light oil) substitutes. On the other hand, the species of the genus Hematococcus, namely, Hematococcus pluvialis or Hematococcus lacustris, can produce astaxanthin which is an antioxidative substance.

As described above, in the algae cultivation method according to the present invention, it is preferred to switch the red light irradiation step and the blue light irradiation step to each other at a time interval suited to the cell division cycle of the subject algae. Specifically, in the case of Botryococcus braunii having a long cell division cycle, a single irradiation cycle comprises, for example, 12 hours of the red light irradiation step and 12 hours of the blue light irradiation step. On the other hand, in the case of green algae of the genus Chlorella having a short cell division cycle, a single irradiation cycle comprises, for example, each 0.1 to 3 hours of the red light irradiation step and the blue light irradiation step.

### Examples

### <Test Example 1:Botryococcus braunii proliferation promoting test>

In this Test Example, Botryococcus braunii which is hydrocarbon-producing algae and a species of green algae was employed to examine the proliferation promoting effect of the alternate irradiation with the red light and the blue light.

Botryococcus braunii strain N-2199 contributed by National Institute for Environmental Studies was subjected to an initial proliferation in an agar culture medium (Hyponex, 1000-fold dilution, 1% agarose). The initial proliferation was conducted in a fluorescent lamp illumination environment. The colonies were picked up from the agar culture medium, combined with 70 µl of distilled water to form a suspension, each 30 µl of which was inoculated to the agar culture medium.

The light sources employed were a red LED (center wavelength: 660nm, produced by Showa Denko K.K.), a blue LED (center wavelength: 480nm, produced by Showa Denko K.K.), and a fluorescent lamp. The number of mounts on a single set of each LED was 240 for both of the red LED and the blue LED.

The experiment groups in the following illumination environments were provided, and cultivated for 3 weeks to form colonies.
"Control group"
   Light source: fluorescent lamp, illuminative light's photosynthetic photon flux density: 140 µmol/m²s, 12-hour light period/12-hour dark period
"LED group"
   Light source: red LED and blue LED, illuminative light's photosynthetic photon flux density: red 87.5, blue 52.5 µmol/m²s (red:blue ratio, 5:3), 12-hour red/12-hour blue (red and blue alternate irradiation)

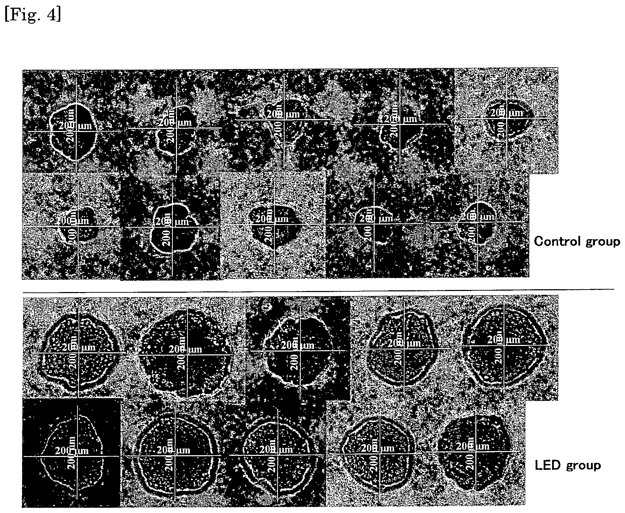

The results are shown in Fig. 4. The upper photograph represents the control group and the lower photograph represents the LED group, each photograph showing 10 colonies selected randomly from the cultivated plate. For the purpose of comparing the colony sizes, the photograph includes a 200 µm scale bar.

The LED group exhibited a larger colony size when compared with the control group. Based on the observation of individual cells, the increase in the colony size was considered to be attributable to the increase in the number of the cells rather than the increase in the size of a cell itself.

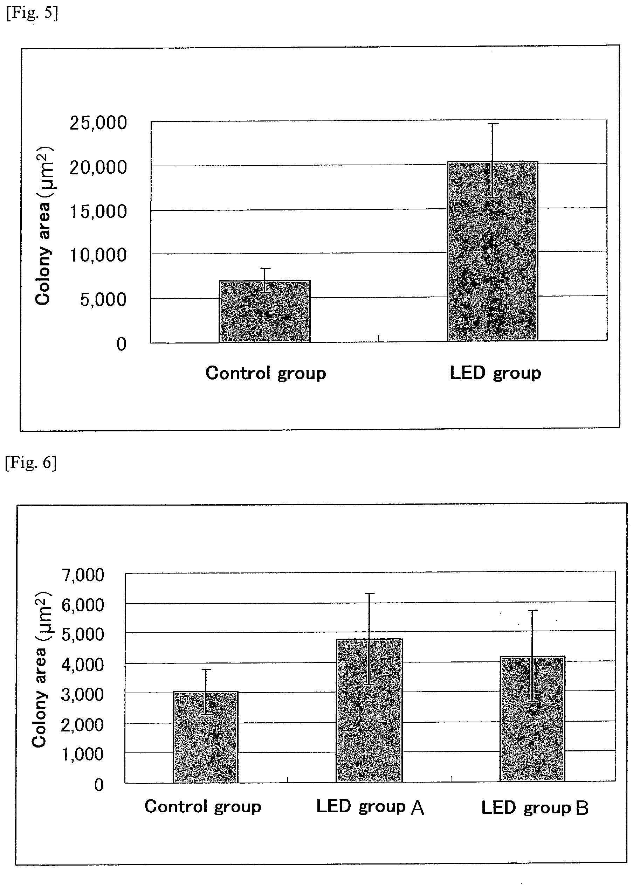

Lenaraf 202b software (Atelier M&M) was employed to measure the colony area and the results are shown in Fig. 5 and Table 1. Fig. 5 is a graph of the mean of the areas of 10 colonies, and the ordinate represents the mean and the standard deviation of the colony areas (µm²). In the LED group, the colony proliferation was more favorable when compared with the control group, and the proliferation was promoted up to about 3 times when compared with the control group within the 3-week cultivation period.

**[Table 1]**

| | Mean | Standard deviation | Maximum | Minimum | Median |
|---|---|---|---|---|---|
| Control group | 7,013 | 1,438 | 9,223 | 5,489 | 6,603 |
| LED group | 20,311 | 4,202 | 30,527 | 17,018 | 18,245 |

| | | | | | |
|---|---|---|---|---|---|
| (In the table, values other than the standard deviations represent the colony areas (µm²)) | | | | | |

Next, the experiment groups in the following illumination environments were provided, and cultivated for 2 weeks to form colonies. The cultivation was conducted by the method described above except for changing the period.
"Control group"
   Light source: fluorescent lamp, illuminative light's photosynthetic photon flux density: 140 µmol/m²s, 12-hour light period/12-hour dark period
"LED group A"
   Light source: red LED and blue LED, illuminative light's photosynthetic photon flux density: red 87.5, blue 52.5 µmol/m²s (red:blue ratio, 5:3), 12-hour red/12-hour blue (red and blue alternate irradiation)
"LED group B"
   Light source: red LED and blue LED, illuminative light's photosynthetic photon flux density: red 87.5, blue 52.5 µmol/m²s (red:blue ratio, 5:3), 12-hour light period/12-hour dark period (red and blue simultaneous irradiation)

The results are shown in Fig. 6 and Table 1. The figure is a graph of the mean of the areas of 10 colonies, and the ordinate represents the mean and the standard deviation of the colony areas (µm²). The LED group A (red and blue alternate irradiation) exhibited a more favorable colony proliferation when compared with the control group and the LED group B (red and blue simultaneous irradiation), and exhibited the fastest proliferation.

**[Table 2]**

| | Mean | Standard deviation | Maximum | Minimum | Median |
|---|---|---|---|---|---|
| Control group | 3,033 | 770.8 | 4,332 | 1,862 | 2,914 |
| LED group A | 4,772 | 1,531 | 7,659 | 2,217 | 4,799 |
| LED group B | 4,161 | 1,530 | 7,271 | 2,242 | 4,099 |

| | | | | | |
|---|---|---|---|---|---|
| (In the table, values other than the standard deviations represent the colony areas (µm²)) | | | | | |

As described above, the results of this Test Example indicated that, when compared with the fluorescent light illumination environment (control group) and the simultaneous illumination environment (LED group B) where the 12-hour simultaneous irradiation with the red light and the blue light and 12-hour dark period were repeated, the alternate irradiation environment (LED group A) where the red light and the blue light were irradiate for each 12 hours alternately resulted in a marked promotion of the cell proliferation. Also in Fig. 4, the oil drops were identified in the colonies in the alternate irradiation environment (LED group A), suggesting that the alternate irradiation promoted not only the cell division but also the hydrocarbon production.

### <Test Example 2: Chlorella kessleri proliferation promoting test>

In this Test Example, Chlorella kessleri, a species of green algae of the genus Chlorella, which is employed widely as algae for experiments and applied also to supplements, was employed to examine the proliferation promoting effect of the alternate irradiation with the red light and the blue light.

Chlorella kessleri C531 strain (identical to Chlorella kessleri strain NIES-2160 possessed by National Institute for Environmental Studies) was subjected to an initial proliferation in an agar culture medium (Hyponex, 1000-fold dilution, 1% agarose). The initial proliferation was conducted in a fluorescent lamp illumination environment. The colonies were picked up from the agar culture medium, combined with 50 µl of distilled water to form a suspension, each 9 µl of which was inoculated to 10 ml of a liquid culture medium (Hyponex, 1000-fold dilution).

The light sources employed were a red LED (center wavelength: 660nm, produced by Showa Denko K.K.), a blue LED (center wavelength: 480nm, produced by Showa Denko K.K.), and a fluorescent lamp. The number of mounts on a single set of each LED was 240 for both of the red LED and the blue LED.

The experiment groups in the following illumination environments were provided, and subjected to the 6-day static cultivation to form colonies.
"Control group"
   Light source: fluorescent lamp, illuminative light's photosynthetic photon flux density: 140 µmol/m²s, 12-hour light period/12-hour dark period
"LED group A"
   Light source: red LED and blue LED, illuminative light's photosynthetic photon flux density: red 105, blue 35 µmol/m²s (red:blue ratio, 3:1), 12-hour light period/12-hour dark period (red and blue simultaneous irradiation)
"LED group B"
   Light source: red LED and blue LED, illuminative light's photosynthetic photon flux density: red 105, blue 35 µmol/m²s (red:blue ratio, 3:1), 12-hour red/12-hour blue (red and blue alternate irradiation)
"LED group C"
   Light source: red LED and blue LED, illuminative light's photosynthetic photon flux density: red 105, blue 35 µmol/m²s (red:blue ratio, 3:1), 3-hour red/3-hour blue (red and blue alternate irradiation)
"LED group D"
   Light source: red LED and blue LED, illuminative light's photosynthetic photon flux density: red 105, blue 35 µmol/m²s (red:blue ratio, 3:1), 0.1-hour red/0.1-hour blue (red and blue alternate irradiation)

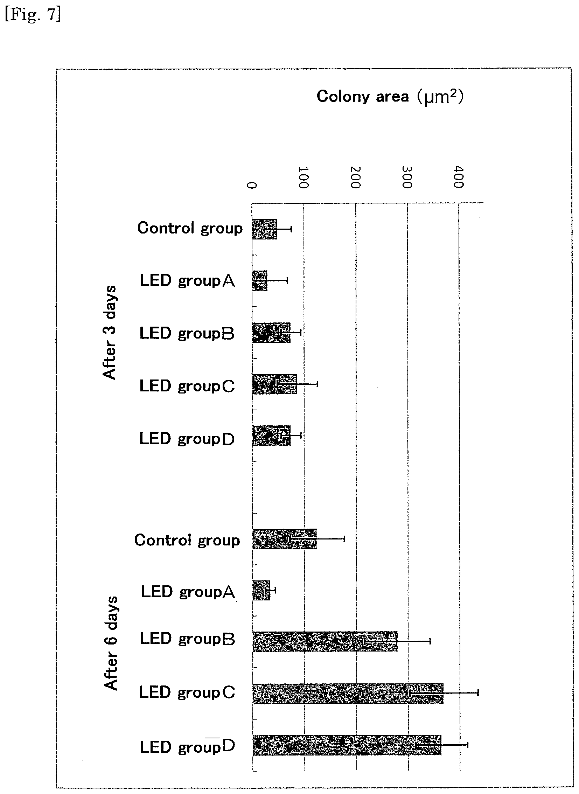

3 Days and 6 days after starting the cultivation, 5 µl was taken out of the cultivation fluid and the cell density (cells/µl) was measured using Thoma hemocytometer. The results are shown in Fig. 7. The ordinate represents the cell density (cells/µl) and the abscissa represents the experiment group. The cell density was represented as a mean of 4 zones of the hemocytometer together with the standard deviation.

No significant difference was observed after 3 days between the cell density in the control group and the cell densities in the LED groups A to D. After 6 days, however, the density in the control group was 125 cells/µl, while that in the LED group B conducting 12-hour alternate irradiation was 280 cells/µl, that in the LED group C conducting 3-hour alternate irradiation was 370 cells/µl, and that in the LED group D conducting 0.1-hour alternate irradiation was 365 cells/µl, showing the proliferation in the experiment groups conducting red and blue alternate irradiation about 2 to 3 times that in the control group.

Based on the preliminary cultivation experiment, it was assumed that Chlorella kessleri exhibits an extremely favorable proliferation in a liquid culture medium (Hyponex, 1000-fold dilution), and the cell division cycle of Chlorella kessleri is shorter than the cell division cycle of Botryococcus braunii. Also in this Test Example, the proliferation effect in Chlorella kessleri was higher in the LED group C conducting 3-hour alternate irradiation than in the LED group B conducting 12-hour alternate irradiation, suggesting the significance of constructing the alternate irradiation cycle in harmony with the cell division cycle.

### <Test Example 3:Hematococcus lacustris proliferation promoting test>

In this Test Example, Hematococcus lacustris which is a species of green algae that produces astaxanthin which is utilized for fish color improvement and in cosmetics and antioxidative supplements was employed to examine the proliferation promoting effect of the alternate irradiation with the red light and the blue light.

Hematococcus lacustris strain NIES-144 contributed by National Institute for Environmental Studies was subjected to an initial proliferation in an agar culture medium (Hyponex, 1000-fold dilution, 1% agarose). The initial proliferation was conducted in a fluorescent lamp illumination environment. The colonies were picked up from the agar culture medium, combined with 600 µl of a liquid culture medium (Hyponex, 1000-fold dilution) to form a suspension, which is cultivated in the fluorescent lamp illumination environment. Thereafter, each 200 µl of the cultivation fluid was inoculated to an agar culture medium.

The light sources employed were a red LED (center wavelength: 660nm, produced by Showa Denko K.K.), a blue LED (center wavelength: 480nm, produced by Showa Denko K.K.), and a fluorescent lamp. The number of mounts on a single set of each LED was 240 for both of the red LED and the blue LED.

The experiment groups in the following illumination environments were provided, and cultivated for 1 week to form colonies.
"Control group"
   Light source: fluorescent lamp, illuminative light's photosynthetic photon flux density: 140 µmol/m²s, 12-hour light period/12-hour dark period
"LED group A"
   Light source: red LED and blue LED, illuminative light's photosynthetic photon flux density: red 105, blue 35 µmol/m²s (red:blue ratio, 3:1), 12-hour light period/12-hour dark period (red and blue simultaneous irradiation)
"LED group B"
   Light source: red LED and blue LED, illuminative light's photosynthetic photon flux density: red 105, blue 35 µmol/m²s (red:blue ratio, 3:1), 12-hour red/12-hour blue (red and blue alternate irradiation)

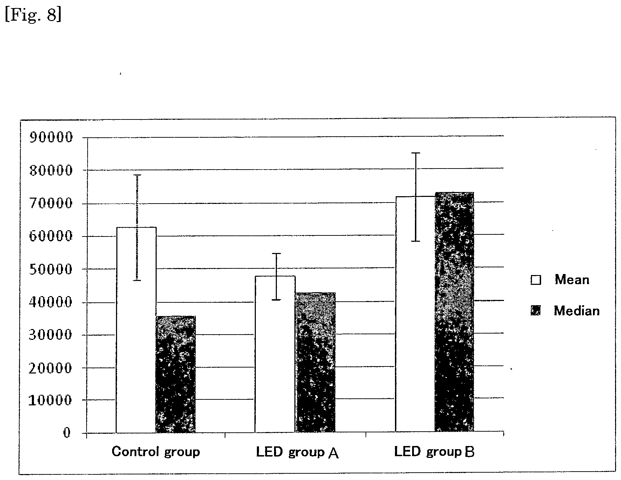

The results of the measurement of the colony areas conducted similarly to Test Example 1 are shown in Fig. 8 and Table 3. The figure is a graph of the mean and the median of the areas of 20 colonies, and the ordinate represents the mean of the colony areas (µm²) together with the standard deviation and the median thereof. In LED group B, the colony proliferation was promoted when compared with the control group.

**[Table 3]**

| | Mean | Standard deviation | Maximum | Minimum | Median |
|---|---|---|---|---|---|
| Control group | 62,665 | 70,140 | 283,541 | 8,349 | 35,704 |
| LED group A | 47,673 | 32,089 | 142,077 | 9,309 | 42,586 |
| LED group B | 71,645 | 61,276 | 225,571 | 7,817 | 73,000 |

| | | | | | |
|---|---|---|---|---|---|
| (In the table, values other than the standard deviations represent the colony areas (µm²)) | | | | | |

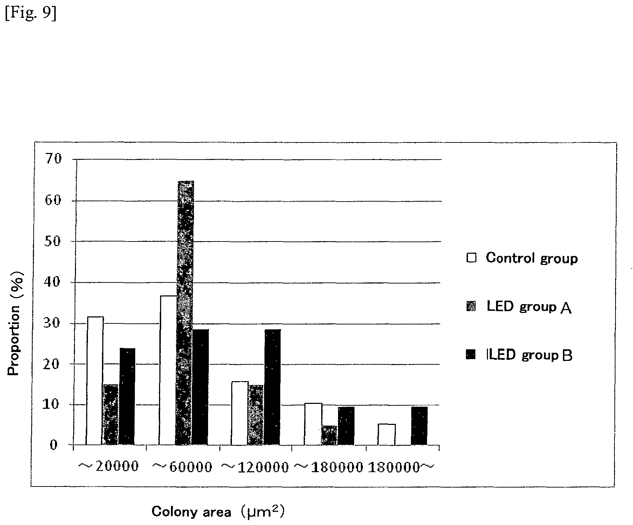

The frequency distribution of the colony area in each experiment group is shown in Fig. 9. In the figure, the number of the colonies contained in each interval of the measured colony area consisting of less than 20,000, 20,000 to 60,000, 60,000 to 120,000, 120,000 to 180,000, 180,000 or more (in µm²) was represented as a proportion. The ordinate represents the proportion (%).

In the control group, those of 20,000 µm² or less in size corresponded to 31.6% and those of 20,000 to 60,000 µm² corresponded to 36.8%, thus those less than 60,000 occupying about 70%. On the contrary, in the LED group B conducting the red and blue alternate irradiation, those of 60,000 to 120,000 corresponded to about 30%, those of 120,000 to 180,000 corresponded to about 10%, and those of 180,000 or more corresponded to about 10%, thus those of 60,000 or more occupying about 50%.

Based on the results of this Test Example described above, it was revealed that also in Hematococcus lacustris the alternate irradiation with the red light and the blue light promotes the cell proliferation markedly.

### Industrial Applicability

According to the algae cultivation method and the like according to the present invention, the proliferation of algae can be promoted by a convenient method thereby shortening the cultivation period and increasing the producibility. As a result, the algae cultivation method and the like according to the present invention can preferably be employed in the cultivation of algae targeted to the starting materials for biological fuels, health foods, and pharmaceuticals.

### Reference Signs List

- S₁:: Red light irradiation step
- S₂:: Blue light irradiation step
- S₃:: Simultaneous irradiation step
- S₄:: Interruption step
- C₁, C₂:: Cycle

## Claims

1. An algae cultivation method for promoting the proliferation of algae belonging to Chlorophyceae, said method comprising conducting an irradiation cycle of alternately and successively carrying out a step of red light irradiation to the algae and a step of blue light irradiation to the algae, each step employing a time period of at least a minute, and repeating said cycle.

2. The algae cultivation method according to Claim 1 wherein the algae belonging to Chlorophyceae are green algae of Botryococcus genus or Chlorella genus.

3. The algae cultivation method according to Claim 1 wherein the algae belonging to Chlorophyceae are green algae of Hematococcus genus.

## Patentansprüche

1. Algenkultivierungsverfahren zur Förderung der Proliferation von zu Chlorophyceae gehörenden Algen, wobei das Verfahren die Durchführung eines Bestrahlungszyklus umfasst, bei dem abwechselnd und nacheinander ein Schritt der Rotlichtbestrahlung der Algen und ein Schritt der Blaulichtbestrahlung der Algen durchgeführt wird, wobei jeder Schritt eine Zeitspanne von mindestens einer Minute in Anspruch nimmt, und der Zyklus wiederholt wird.

2. Algenkultivierungsverfahren nach Anspruch 1, wobei die zu Chlorophyceae gehörenden Algen Grünalgen der Gattung Botryococcus oder Chlorella sind.

3. Algenkultivierungsverfahren nach Anspruch 1, wobei die zu Chlorophyceae gehörenden Algen Grünalgen der Gattung Hematococcus sind.

## Revendications

1. Procédé de culture d'algues pour induire la prolifération d'algues appartenant aux chlorophycées, ledit procédé comprenant l'exécution d'un cycle d'irradiation consistant à réaliser alternativement et successivement une étape d'exposition des algues à une lumière rouge et une étape d'exposition des algues à une lumière bleue, chaque étape employant une période de temps d'au moins une minute, et la répétition dudit cycle.

2. Procédé de culture d'algues selon la revendication 1 dans lequel les algues appartenant aux chlorophycées sont des algues vertes du genre Botryococcus ou du genre Chlorella.

3. Procédé de culture d'algues selon la revendication 1 dans lequel les algues appartenant aux chlorophycées sont des algues vertes du genre Hematococcus.
